# EUROPEAN PATENT APPLICATION

(11) **EP 3 846 177 A1**
(43) Date of publication of application: **07.07.2021**
(21) Application number: 20150174.9
(22) Date of filing: 03.01.2020
(51) Int. Cl.: G16H 40/60, G16H 80/00

(54) **AN INTERACTIVE USER SYSTEM AND METHOD**

(71) Applicant: Koninklijke Philips N.V., 5656 AG Eindhoven (NL)
(72) Inventor: BULUT, Murtaza, 5656 AE Eindhoven (NL); HILBIG, Rainer, 5656 AE Eindhoven (NL); SHI, Jun, 5656 AE Eindhoven (NL); VAN DER ZAAN-LANDWEHR JOHAN, Suzanne Daniëlle, 5656 AE Eindhoven (NL); ZHANG, Qiushi, 5656 AE Eindhoven (NL)
(74) Representative: Philips Intellectual Property & Standards

(57) **Abstract**

The invention provides an interactive user system adapted to adjust the manner in which a system interacts with a target user and a support user, the support user being a support provider of the target user. The system includes a sensor arrangement comprising one or more sensors for monitoring the target user and/or the support user and/or receiving an input from the target user and/or the support user, thereby obtaining user data relating to a mental state of the target user and/or the support user, for example a behavioral state or a psychological state of the target user and/or the support user.

The system further includes a processor adapted to: operate the interactive user system in an initial operation mode, the initial operation mode having a mode type; determine, based on the user data and the mode type, an operation mode adjustment; and apply the operation mode adjustment to the interactive user system.

## Description

### FIELD OF THE INVENTION

The invention relates to the field of interactive user systems, and more specifically to the field of automatic operation mode adjustment.

### BACKGROUND OF THE INVENTION

During times of stress a person may undergo significant variations in their emotions and moods. For example, during a pregnancy hormone level (and hence the resulting physiological and psychological changes) are highly variable and therefore unpredictable and changes in the first, second and third trimesters are all different.

Mood swings are common during times of stress, and the effect of the mood swings on the state of the person and the people around them can be significant. The unpredictable nature of these mood swings can make them difficult to cope with and can have significant detrimental effects. Some examples of emotions and moods dominating different states of stress are: anxiety, fear, forgetfulness, and sensitivity.

Ideally interactions with the person undergoing the stress should take into account the potential changes in their physical and mental state, and should adapt accordingly.

The psychological and physiological state of person undergoing significant stress can change on a daily (or sometimes hourly) basis. Such changes necessitate different kinds of monitoring and interventions (i.e., engagement and communication).

Existing solutions are based on monitoring the person by objective (using sensors) or subjective (based on user input) means. Usually, an intervention is employed and a user's reactions to the intervention is monitored, and the future interventions are adapted according to the collected user data.

The main limitation of these solutions are that they are not suitable to predict and cope with the highly unpredictable nature of mood and emotion fluctuations. Moreover, they are not able to accurately model the effect of the changes on the users' physiological and psychological state, because of potential user non-compliance (for example, a user may not respond to a question) and hence a lack of sufficient and suitable monitoring data for these periods. Arguably, for monitoring of these periods, the most valuable data is the subjective user input data; however, it is the most likely that the user will not want to answer any questionnaires during these periods.

There is therefore a need to provide a means of improved user support.

### SUMMARY OF THE INVENTION

The invention is defined by the claims.

According to examples in accordance with an aspect of the invention, there is provided an interactive user system adapted to adjust the manner in which a system interacts with a target user and a support user, the support user being a support provider of the target user, the system comprising:
a sensor arrangement comprising one or more sensors for monitoring the target user and/or the support user and/or receiving an input from the target user and/or the support user, thereby obtaining user data relating to a mental state of the target user and/or the support user, for example a behavioral state or a psychological state of the target user and/or the support user; and
a processor adapted to:
   operate the interactive user system in an initial operation mode, the initial operation mode having a mode type;
   determine, based on the user data and the mode type, an operation mode adjustment; and
   apply the operation mode adjustment to the interactive user system.

The system provides a means of intelligently adjusting the operation of the interactive user system based on user data obtained by monitoring the target user and support user and/or by receiving an input from one or both of the users.

The system provides a means of adjusting a function of the system based on the obtained user data, for instance, an alert indicating an elevated stress level of the target user based on the user data, such as an increased heart rate or the detection of a raised voice. Further, it may be determined that the target user is not in a receptive state based on the user data, in which case the stress alert may be provided to the support user, who may then interact with the target user to address the issue.

By providing an intelligent operation adjustment, the interactive user system may be operated in a manner best suited to the target user and/or the support user.

In an embodiment, the operation mode comprises a sensor arrangement operation mode, and wherein determining the operation mode adjustment comprises:
determining a monitoring scheme based on the user data, wherein the monitoring scheme comprises:
   selecting the target user and/or the support user for monitoring;
   selecting one or more sensors of the sensor arrangement for obtaining further user data from the user selected for monitoring; and
applying the monitoring scheme to the sensor arrangement.

In this way, the monitoring of the target user and/or the support user may be adjusted according the current state of one or both of the users, thereby increasing the likelihood that the user data obtained will be relevant to the current user state.

In a further embodiment, selecting one or more sensors of the sensor arrangement comprises selecting a first set of the one or more sensors for monitoring the target user and a second set of the one or more sensors for monitoring the support user

In this way, the monitoring may be tailored to each of the users, thereby increasing the likelihood that the user data obtained will be relevant to the current user state of each individual user.

In an embodiment, the operation mode comprises a user data evaluation mode, and wherein determining the operation mode adjustment comprises:
identifying the initial operation mode, which comprises a preliminary user data evaluation mode for processing the user data;
generating an evaluation mode adjustment based on the user data; and
applying the evaluation mode adjustment to the preliminary user data evaluation mode, thereby generating an adjusted user data evaluation mode.

In this way, the manner in which the user data is interpreted or processed may be adjusted based on the current state of one or both of the users, thereby increasing the likelihood that the user data will be interpreted in a manner that is relevant to the current user state.

In an embodiment, the initial operation mode comprises a preliminary interaction mode, and wherein determining the operation mode adjustment comprises:
identifying an interaction type of the preliminary interaction mode;
determining, based on the user data and the interaction type, whether the preliminary interaction mode is to be received by the target user and/or the support user; and
adjust the preliminary interaction mode based on the determination of a recipient user and the interaction type, thereby generating an adjusted interaction mode, and wherein:
   the system further comprises a user interface adapted to interact with the recipient user using the adjusted interaction mode.

In this way, the system may adapt a user interaction based on the user data in order to interact with one or both of the users in an optimal manner.

In an embodiment, the system is adapted to adjust the manner in which a system interacts with a plurality of target users and a support user, and wherein determining the operation mode adjustment comprises:
for each of the plurality of target users, determining a target user priority for receiving support from the support user; and
determining the operation mode adjustment based on the plurality of target user priorities.

In this way, the system may account for a given support user providing support to multiple target users.

In an embodiment, the system is adapted to adjust the manner in which a system interacts with a target user and a plurality of support users, and wherein determining the operation mode adjustment comprises:
for each of the plurality of support users, determining a support user suitability score for providing support to the target user; and
determining the operation mode adjustment based on the plurality of support user suitability scores.

In this way, the system may interact with the support user best suited to address a given issue of the target user based on the user data.

In an embodiment, determining the operation mode adjustment is performed using a machine learning algorithm.

In this way, the system may adapt to a given target user and/or support user over time.

In an embodiment, the system further comprises a memory adapted to store historic user data relating to the target user and/or the support user, and wherein determining the operation mode adjustment is further based on the historic user data.

In this way, the system may refer to previous user interactions in order to guide the adjustment of a subsequent system operation adjustment.

In a further embodiment, the historic user data comprises user feedback relating to a subjective user experience based on the adjusted operation mode.

By incorporating subjective user feedback, the system may be further adapted to adjust the interaction delivery and content based on the preferences of the target user and/or the support user.

In an embodiment, the memory is further adapted to store a user characteristic relating to the target user and/or the support user, and wherein determining the operation mode adjustment is further based on the user characteristic.

In this way, a given user characteristic, such as a medical condition, may be taken into account by the system when adjusting the operation of the system.

In an embodiment, the user data comprises one or more of:
pre-operation adjustment user data; and
post-operation adjustment user data.

In this way, the user data may be separated for use in operating the system in an initial operation mode (pre-interaction user data) and for use in gauging a user reaction to the operation adjustment (post-operation adjustment user data).

In an embodiment, the one or more sensors of the sensor arrangement comprises:
a wearable sensor;
an epidermal sensor;
an implantable sensor;
an environmental sensor;
a smart device;
a smartphone;
a smart home device;
a microphone;
a camera;
a thermometer; and
a weight scale.

According to examples in accordance with an aspect of the invention, there is provided a method for adjusting a manner in which a system interacts with a target user and a support user, the support user being a support provider of the target user, the method comprising:
monitoring the target user and/or the support user and/or receiving an input from the target user and/or the support user, thereby obtaining user data relating to a mental state of the target user and/or the support user, for example a behavioral state or a psychological state of the target user and/or the support user;
operating the system in an initial operation mode, the initial operation mode having a mode type;
determining, based on the user data and the mode type, an operation mode type adjustment; and
applying the operation mode adjustment to the interactive user system.

In an embodiment, the method further comprises obtaining user feedback, wherein the user feedback relates to a subjective user experience based on the adjusted operation mode.

According to examples in accordance with an aspect of the invention, there is provided a computer program comprising computer program code means which is adapted, when said computer program is run on a computer, to implement the methods described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a schematic representation of a system according to an aspect of the invention; and
Figure 2 shows a method of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an interactive user system adapted to adjust the manner in which a system interacts with a target user and a support user, the support user being a support provider of the target user. The system includes a sensor arrangement comprising one or more sensors for monitoring the target user and/or the support user and/or receiving an input from the target user and/or the support user, thereby obtaining user data relating to a mental state of the target user and/or the support user, for example a behavioral state or a psychological state of the target user and/or the support user.

The system further includes a processor adapted to: operate the interactive user system in an initial operation mode, the initial operation mode having a mode type; determine, based on the user data and the mode type, an operation mode adjustment; and apply the operation mode adjustment to the interactive user system.

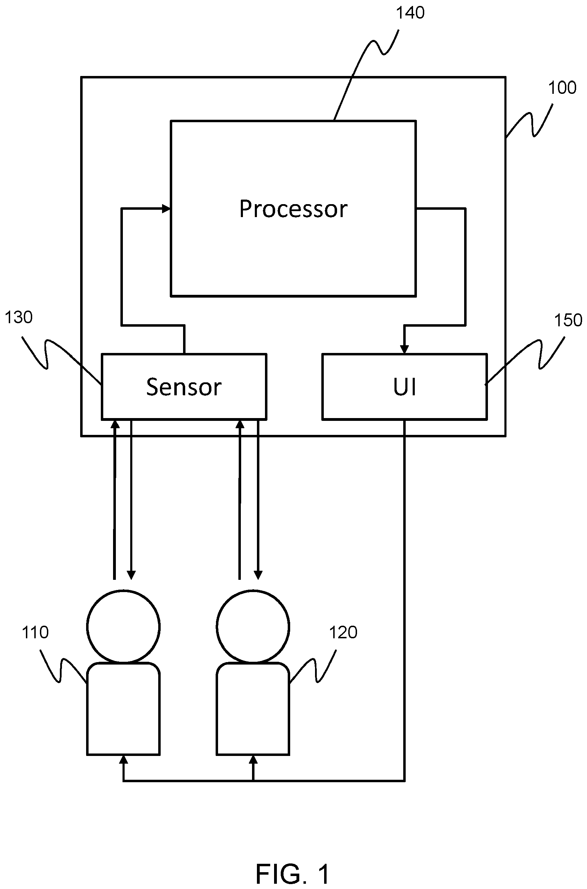

Figure 1 shows an example of an interactive user system 100 adapted to adjust the manner in which a system interacts with a target user 110 and a support user 120, the support user being a care giver of the target user.

The system 100 includes a sensor arrangement 130 for monitoring the target user 110 and/or the support user 120 and/or receiving an input from the target user and/or the support user. The sensor arrangement 130 obtains user data relating to a current mental capacity of the target user, for example a stress level, a behavioral state or a psychological state of the target user and/or the support user.

The functions of the system 100 are described herein in the context of a pregnancy, wherein the target user 110 may be a pregnant woman and the support user 120 may be a partner, or other care giver, of the target user. However, the system may be utilized by any user that may require, or benefit from, a system adapted to adjust the manner in which it engages with the user. For example, the target user 110 may include: a pregnant user; an elderly user; a child; an unwell user; and the like. Put another way, the target user may be any user that has a temporary, or permanent, limitation to their ability to engage with the interactive user system, and therefore may require an adjustable amount of support, which may be in the form of a support user, to facilitate better engagement with the system and monitoring of the target user.

The sensor arrangement 130 may comprise a variety of sensors according to the application of the interactive user system. For example, the senor arrangement may include one or more of: a wearable sensor; an epidermal sensor; an implantable sensor; an environmental sensor; a smart device; a smartphone; a smart home device; a microphone; a camera; a thermometer; a weight scale; and the like.

For example, one or more wearable sensors may be used to monitor the target user 110, and in some cases to monitor the support user 120. Wearable sensors, may be used to monitor user data including one or more of: a heart rate; a heart rate variability; a blood pressure; a skin conductance; a skin temperature; an activity level; a sleep stages; an EEG; a respiration signal; an SP02 signal; a movement signal; and the like. Further, non-wearable sensors may be used to collect user data, wherein such devices may include: a smart weight scale, for monitoring data such as weight, BMI, body posture, and fatigue; a smart mirror, for monitoring skin condition and facial expression; a microphone, for speech monitoring and any other sound monitoring, such as breathing and coughing; and vehicle sensor for monitoring the target user and/or the support user when in a vehicle, such as reaction characteristics, speed and concentration. In addition, smart home sensors may also be used to collect user data, wherein such sensors may include: a microphone, for monitoring environmental sound levels; an air quality sensors; a temperature sensor; a food sensor, for example monitoring gas usage, air fryer sensors, refrigerator sensors, freezer sensors, smart utensils, and the like.

Further, the user data may comprise one or more of: a measure of social activity; a level of interaction with other humans; a level of interaction with a device; data relating to food and/or beverage intake; toilet habits; data relating to travel behaviour; data relating to medication intake; and the like.

It should be noted that the examples of sensors provided above are provided as examples only. The provided list of sensors is not exhaustive and is provided for the purpose of illustration.

The system 100 further includes a processor 140 in communication with the sensor arrangement 130.

The processor 140 is adapted to operate the interactive user system in an initial interaction mode. For example, when the initial operation mode is a preliminary interaction mode, in response to the sensor arrangement detecting a change in the health of the target user, the processor may generate a notification containing health related information using the preliminary interaction mode, which may simply be to deliver the notification directly to the target user.

The processor is then adapted to determine an operation mode adjustment based on the user data and adjust the operation mode based on the determined adjustment. In the example of the operation mode being an interaction mode, the preliminary interaction mode has an interaction type denoting the content of the notification. For example, the interaction type may include plain information or may include emotional content, based on interactions determined to be of an emotional nature.

The processor 140 is then adapted to determine, based on the user data and the interaction type, whether the preliminary interaction mode is to be received by the target user and/or the support user. The determination of the recipient user is based performed using a machine learning algorithm.

For example, if the interaction type is plain information without emotional content, the target user may receive the interaction. Alternatively, if the interaction type contains emotional content, and the target user is determined to not be in a receptive mood (such as the target user being in a state of high stress), the support user may be selected as the recipient user.

The processor 140 adjusts the preliminary interaction mode based on the determination of a recipient user and the interaction type, thereby generating an adjusted interaction mode.

For example, an interaction type including information and emotional content may be adjusted to provide only information to the target user and both the information and the emotional content to the support user.

The system 100 may further include a user interface 150 adapted to interact with the recipient user using the adjusted interaction mode. The user interface may include any device capable of providing the adjusted interaction mode to the recipient user, such as a smart device of the user, for example: a smartphone; a personal computer; a laptop; a tablet; a smart watch; a smart home assistant; a smart television; a medication dispenser; a food processor; a massage mat; and the like.

The interaction mode may comprise one or more of: audio or speech based interaction; visual interaction, such as image based or text based interaction; haptic based interaction; olfactory based interaction; or taste based interaction; or any combination of above.

The adjusted interaction mode may include: an adjusted content of the message; an adjusted timing of message delivery; an adjusted medium of message delivery; an adjusted context in which the user should receive the message; and the like. The user interface may be the same user interface or different user interfaces for the target and the support user.

The processor 140 may be adapted to adjust the interaction mode in a number of a ways. For example, the processor may be adapted to select a message from a list of predefined messages or to fill a message template with values calculated from the user data.

Alternatively, the processor 140 may employ a machine learning engine trained using the user data to learn a preferred interaction mode, and to generate content automatically. For example, for text based interactions, the machine learning engine may be trained to establish a connection between the user data and words, from which a natural language generation engine may be used to construct text messages utilizing the selected words.

A machine-learning algorithm is any algorithm that processes input data in order to produce or predict output data. Here, the input data comprises the user data and the output data comprises the adjusted interaction mode.

Suitable machine-learning algorithms for being employed in the present invention will be apparent to the skilled person. Examples of suitable machine-learning algorithms include decision tree based algorithms and artificial neural networks. Other machine-learning algorithms such as deep learning, logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example user data. The training output data entries correspond to adjustments to the interaction mode.

The machine learning engine may be trained in a supervised manner, such as with labelled input and output samples, using representative user data. An example set of training data for a pregnant target user is shown in Table 1 below.

**Table 1: Examples of intervention delivery classification based on the intervention content**

| **Content examples** | **Interaction mode** |
|---|---|
| Health of target user, and non-emotional | Deliver to target user |
| Advice to the support user (e.g. support target user during pregnancy by increasing face to face interactions) | Deliver to support user |
| Information about pregnancy stages and fetus growth | Deliver to target user and support user |
| Emotional content (e.g. potential health issue) | Deliver to target user via support user |

The example above shows that delivery classification, i.e. the determination of the recipient user, may be performed using a supervised approach where particular content categories have been labelled with the delivery classification labels.

Further, a complimentary way to achieve the delivery classification is to use the user data that has been collected by the sensor arrangement 130. This is exemplified in Table 2 below.

**Table 2: Examples of intervention delivery classification based on past user data.**

| **User data characteristics examples** | **Delivery classification** |
|---|---|
| Similar content did not result in significant changes in the heart rate variability and skin conductance of the target user | Deliver to target user |
| Feedback (collected using questionnaires) from support user showed that they have had a positive reaction to this type of content | Deliver to support user |
| Similar content resulted in significant and similar increase in the activity level of support user and the target user, indicating that they performed the same suggested activity together | Deliver to target user and support user |
| Similar content resulted in increase in stress levels of the target user, and decreased sleep quality | Deliver to target user via support user |

Table 1 and Table 2 provide several examples of the type of information (i.e. user data) that may be used to train the machine learning engine.

In addition, the processor 140 may be adapted to control the user interface 150 to prompt the target user 110 and/or the support user 120 to provide user feedback, wherein the user feedback relates to a subjective user experience based on the adjusted interaction mode.

In this way, the user data may include both objective user data, obtained from the sensor arrangement, and subjective user data, which may be collected by asking the users to provide a user input, for example using questionnaires, or an open input.

The system may further comprise a memory adapted to store historic user data relating to the target user and/or the support user, and wherein generating the preliminary interaction mode, determining the recipient user and adjusting the preliminary interaction mode is further based on the historic user data. The user feedback may form part of the historic user data. In addition, the memory may be further adapted to store a user characteristic relating to the target user and/or the support user, and wherein generating the preliminary interaction mode, determining the recipient user and adjusting the preliminary interaction mode is further based on the user characteristic. The user characteristic may be any information relating to the target user or the support user relevant to the interaction mode of the system 100.

The processor 140 may be further adapted to select one or more of the sensors for monitoring the target user and/or the support user based on the user data. Different sets of sensors may be selected for the target user and the support user.

Similar to the delivery classification above, the operation mode may comprise a sensor arrangement operation mode, which may be used to select monitoring options for the target user and the support user, which may include: monitor the target user, using sensors and questionnaires; monitor the support user, using sensors and questionnaire; monitor the target user and the support user, using sensors and questionnaires; and monitor the target user via the support user, using unobtrusive and ubiquitous sensors to monitor the target user, and prompting the support user to provide input about the target user, thereby minimizing the disturbance to, and involvement of, the target user.

Some examples of how the machine learning engine can be trained to select a monitoring option are given below in Table 3.

**Table 3: Examples showing deriving the monitoring options based on the intervention content features.**

| **Content Examples** | **Monitoring options** |
|---|---|
| Content related to relaxation (e.g. breathing) exercises that can be performed | Monitor target user |
| Content related to the changes expected in target user body and behavior during pregnancy (aiming increasing awareness of the partner with regard to mood swings for example) | Monitor support user |
| Healthy eating advice | Monitor target user and support user |
| Questions related to the psychological (e.g. affective) state of target user | Monitor target user via support user |

The monitoring options may be also selected based on the historical user data, examples of which are shown in Table 4 below.

**Table 4: Examples showing deriving the monitoring options based on the intervention content and sensor data.**

| **Content examples and sensor data** | **Monitoring options** |
|---|---|
| Data show that target user wears the activity monitor continuously & content related to activity monitoring | Monitor target user |
| Data show that support user is the one preparing the meals & content related to healthy eating | Monitor support user |
| Data show that target user and support user have different sleeping patterns, but sleep in the same bed & content related to sleep | Monitor target user and support user |
| Data show that sensor data cannot be used to calculate stress level and emotional state of target user | Monitor target user via support user |

In addition, the devices and sensors may be determined based on the content analysis of the interaction mode. For example, the content of the interaction mode may be classified by categories, such as food, activity, sleep, stress and the like, and only the sensors that collect user data related to the desired category may be engaged.

In addition, selecting which sensors are to be uses may be based on the intervention content, user data collection and analysis, which may be further tuned by adapting the corresponding sensor settings of the sensor arrangement. For example, certain user data content may require that particular sensors are operated with a higher sampling frequency, and for a longer duration of time. In a specific example, when the user data comprises emotional content, because the effect of such content on the target user may be unpredictable, a PPG sensor for example may be operated with a higher sampling frequency and for longer duration of time so that heart rate variability can be calculated, for which the processor resources may be allocated to make the calculation in almost real-time.

In addition to adjusting the monitoring scheme of the sensor arrangement, the operation mode adjustment may be used to adjust the manner in which the user data is evaluated. In particular, the processor may be adapted to identify the initial operation mode, which comprises a preliminary user data evaluation mode for processing the user data and generate an evaluation mode adjustment based on the user data. The evaluation mode adjustment is them applied to the preliminary user data evaluation mode, thereby generating an adjusted user data evaluation mode.

As it is described above, the pre-interaction monitoring options, used to collect the user data, are selected and the target user and/or the support user are monitored accordingly. Using the collected user data, the message delivery classification (as described above) may be verified. If the verification is successful, in other words, if the collected user data indicates that the selected interaction mode is appropriate, then the intervention can be realized. If the collected pre-message data indicates that the previously selected interaction mode is not suitable, then the whole process may be repeated from the beginning (i.e. the steps of message generation, classification, and monitoring are repeated).

Using the machine learning engine described above a link between interaction features, user data, and delivery options may be established. In others words, for a given interaction mode and user data, the trained machine learning engine outputs a suitable (adjusted) interaction mode.

The processor 140 may also verify if an adjustment to an interaction mode done before (which was based on the historic user data) is still valid. Using new user data (collected during pre-message monitoring by the sensor arrangement 130) a new adjustment for the message delivery classification type may be generated (using the machine learning engine trained on the past user data). If the output adjustment matches the previous output adjustment (which was generated before the pre-message monitoring user data was available) then the verification is successful. If not, the process may be repeated from the beginning.

The characteristics of the intervention mode to be delivered to the target user 110 or the support user 120 are used to determine the post-message monitoring options. Depending on the content of the user interaction, the sensors of the sensor arrangement 130 that have been selected are activated. In addition, the settings of the selected sensors, such as the sampling frequency, monitoring duration, amount and/or type of stored data are adjusted, or the processing means of the sensor data (for example, using real-time processing, non-real time processing, in a cloud based processing system or in a local processing system) are altered so that more and higher quality user data is collected, or the user data is processed faster.

Another method to determine the post-adjustment monitoring characteristics of the sensor arrangement is to consider the pre-message user data and select what needs to be monitored accordingly. Put another way, pre-operation adjustment user data and interaction type may be analyzed together to determine the post-operation adjustment monitoring options.

Examples of how the content of the interaction may be used to determine the post-message monitoring options and settings, are shown above in Table 3. Additional examples are shown below in Table 5.

**Table 5. Determining the post-intervention monitoring options based on the intervention content characteristics**

| **Intervention content examples** | **Monitoring options** |
|---|---|
| Intervention about physical activity and sleep coaching to be delivered to target user | Utilize wearable physical activity and sleep monitors with higher frequency and accuracy, and utilize the rest of the available and relevant sensors (e.g. social activity monitoring) in a normal manner, or with lower resolution and less functionality (e.g. disable real-time processing, collect less data, etc.) |
| Intervention to be delivered to support user, and it is about support user providing emotional support to target user | Utilize sensors (e.g. speech and video) to monitor affective state of target user, as well as affective state of support user. Monitor the interaction between target user and support user as well. In this case, the monitoring is focused on speech and video, while in most of the cases speech and video sensors may have been disabled due to privacy reasons. |

Examples of how pre-intervention user data may be used to determine post-intervention monitoring and the settings for the selected sensors are shown below in Table 6.

**Table 6. Using pre-intervention sensor data to set the post-intervention monitoring options.**

| **Pre-intervention sensor data** | **Post-intervention sensor data** |
|---|---|
| Irregular patterns (e.g. in sleep behavior). Irregular patterns can be detected by comparing the distribution of the pre-intervention data to the distribution of the historic data. Also, outlier analysis can be performed. | Utilize sleep monitoring sensors, and operate them with higher resolution and accuracy. Also allocate resources so that sleep data can be processed and analyzed in real time. Provide real-time feedback to the user. |
| Regular patterns in the user data (e.g. in daily step count) | Continue monitoring activity as usual, without allocating more resources to the activity monitoring sensors. |
| Changes in eating behavior (in comparison to the historic user data) | Utilize sensors related to eating monitoring, also require the support user to provide more frequent and more detailed feedback (i.e. more detailed and more frequent questionnaires sent to the support user) |

The examples described above have been described in the context of a pregnant target user and a support user.

The system may also be employed between an elderly support user and a support user, such as a younger relative or care giver.

For example, it may be the case that, due to the rapid development in technology, a gap develops between the capabilities of elderly people and the functionalities of the devices they use, making it more difficult for the elderly to understand the content provided to them and to operate the devices.

In this case, the support user 120 may indicate a characteristic of the elderly target user 110. For example, the characteristic may be a physical limitation or a mental limitation. Taking these limitations into account, the interaction modes of the system that are related to the limitations of the elderly target user, may be selected to be delivered via the support user to the elderly target user; whereas, interactions that do not conflict with the indicated limitations may be delivered directly to the elderly target user.

In other words, the selection of the how the interaction is going to be delivered is tuned based on the limitations or attention points of the target user.

For example, if for a particular elderly target user, the user characteristic is that the target user is having occasional lapses in mental capacity (for example, due to disease or medication), then messages that are complex and difficult to understand may be delivered with the help of the support user.

The system 100 may also be used in a medical context, wherein the target user 110 is a subject undergoing treatment or medical monitoring and the support user 120 is a clinician.

For example, in the case of treating a skin condition, the target user 110 is the user with a skin condition and who is using various skin products and participating in skin treatment sessions with professional clinics, and the support user is a clinician helping the target user.

In this case, the sensor arrangement 130 may comprise a smart mirror to capture video data of the target user's skin condition. In addition, the sensors of the devices that are used for the treatment of the target user may also form part of the sensor arrangement.

The communication of the system, i.e. the interaction mode, is mainly directed to the target user. However, in some cases, the interaction mode may be adjusted to deliver an interaction to both the clinician and the target user. An example of such a message is an appointment scheduling messages that requires action from both the clinician and the user. Some types of messages may only be delivered to the target user via the clinician. An example of such a message is a message that is related to progress of the skin disease, which may be loaded with difficult to interpret technical content. In these cases, the target user may receive a video call from the clinician, who can explain the progress.

By distributing the communication of the system between different parties by taking into account user data and interaction characteristics, the system may provide better and more efficient support to the target user.

Rather than a single support user 120, there may be a plurality of support users linked with a target user, for example, the target user being a pregnant woman, the plurality of support users may include the partner and a midwife. In cases where more than one supporting user is involved, the interaction characteristics and historic user data collected from the users may be used to determine the most suitable supporting user that can deliver the information to the target user.

The criteria is that the interaction achieves the desired impact on the target user. An example implementation would be to rank the reaction of the target user with past interventions of the similar characteristics to determine the most suitable supporting user to deliver the current interaction. For example, for interventions that have a high emotional influence on the target user, the partner may be the best candidate (i.e. past data shows that when delivered by the partner, such interactions were well received), while for interventions with technical content, the midwife may be more suitable.

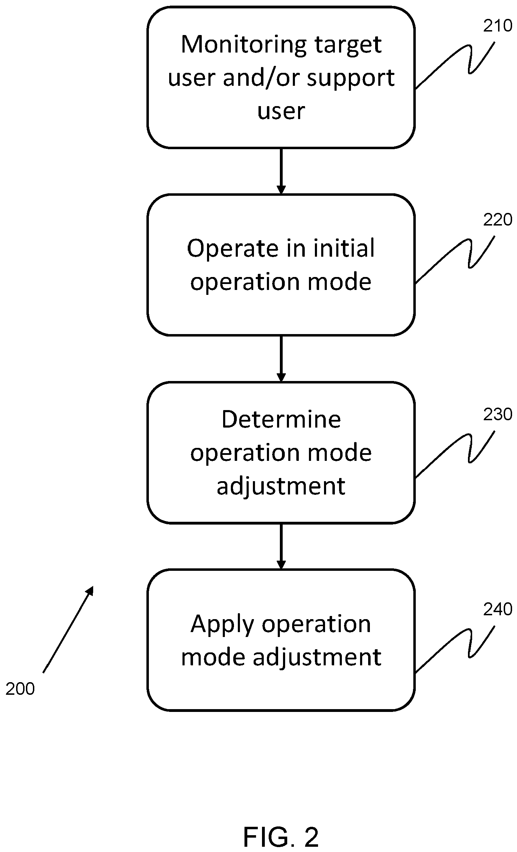

Figure 2 shows a method 200 for adjusting a manner in which an interactive user system interacts with a target user and a support user, the support user being a care giver of the target user.

The method begins in step 210, monitoring the target user and/or the support user and/or receiving an input from the target user and/or the support user, thereby obtaining user data relating to a mental state of the target user and/or the support user, for example a behavioral state or a psychological state of the target user and/or the support user.

In step 220, the system is operated in an initial operation mode, the initial operation mode having a mode type.

It is determined in step 230, based on the user data and the mode type, how the operation mode should be adjusted using an operation mode adjustment.

In step 240, the operation mode adjustment is applied to the interactive user system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An interactive user system (100) adapted to adjust the manner in which a system interacts with a target user (110) and a support user (120), the support user being a support provider of the target user, the system comprising:
a sensor arrangement (130) comprising one or more sensors for monitoring the target user and/or the support user and/or receiving an input from the target user and/or the support user, thereby obtaining user data relating to a mental state of the target user and/or the support user, for example a behavioral state or a psychological state of the target user and/or the support user; and
a processor (140) adapted to:
operate the interactive user system in an initial operation mode, the initial operation mode having a mode type;
determine, based on the user data and the mode type, an operation mode adjustment; and
apply the operation mode adjustment to the interactive user system.

2. A system (100) as claimed in claim 1, wherein the operation mode comprises a sensor arrangement operation mode, and wherein determining the operation mode adjustment comprises:
determining a monitoring scheme based on the user data, wherein the monitoring scheme comprises:
selecting the target user and/or the support user for monitoring;
selecting one or more sensors of the sensor arrangement for obtaining further user data from the user selected for monitoring; and
applying the monitoring scheme to the sensor arrangement.

3. A system (100) as claimed in claim 2, wherein selecting one or more sensors of the sensor arrangement comprises selecting a first set of the one or more sensors for monitoring the target user and a second set of the one or more sensors for monitoring the support user

4. A system (100) as claimed in any of claims 1 to 3, wherein the operation mode comprises a user data evaluation mode, and wherein determining the operation mode adjustment comprises:
identifying the initial operation mode, which comprises a preliminary user data evaluation mode for processing the user data;
generating an evaluation mode adjustment based on the user data; and
applying the evaluation mode adjustment to the preliminary user data evaluation mode, thereby generating an adjusted user data evaluation mode.

5. A system (100) as claimed in any of claims 1 to 4, wherein the initial operation mode comprises a preliminary interaction mode, and wherein determining the operation mode adjustment comprises:
identifying an interaction type of the preliminary interaction mode;
determining, based on the user data and the interaction type, whether the preliminary interaction mode is to be received by the target user and/or the support user; and
adjust the preliminary interaction mode based on the determination of a recipient user and the interaction type, thereby generating an adjusted interaction mode, and wherein:
the system further comprises a user interface (150) adapted to interact with the recipient user using the adjusted interaction mode.

6. A system (100) vas claimed in any of claims 1 to 5, wherein the system is adapted to adjust the manner in which a system interacts with a plurality of target users and a support user, and wherein determining the operation mode adjustment comprises:
for each of the plurality of target users, determining a target user priority for receiving support from the support user; and
determining the operation mode adjustment based on the plurality of target user priorities.

7. A system (100) as claimed in any of claims 1 to 5, wherein the system is adapted to adjust the manner in which a system interacts with a target user and a plurality of support users, and wherein determining the operation mode adjustment comprises:
for each of the plurality of support users, determining a support user suitability score for providing support to the target user; and
determining the operation mode adjustment based on the plurality of support user suitability scores.

8. A system (100) as claimed in any of claims 1 to 7, wherein determining the operation mode adjustment is performed using a machine learning algorithm.

9. A system (100) as claimed in any of claims 1 to 8, wherein the system further comprises a memory adapted to store historic user data relating to the target user and/or the support user, and wherein determining the operation mode adjustment is further based on the historic user data.

10. A system (100) as claimed in claim 9, wherein the historic user data comprises user feedback relating to a subjective user experience based on the adjusted operation mode.

11. A system (100) as claimed in any of claims 9 to 10, wherein the memory is further adapted to store a user characteristic relating to the target user and/or the support user, and wherein determining the operation mode adjustment is further based on the user characteristic.

12. A system (100) as claimed in any of claims 1 to 11, wherein the user data comprises one or more of:
pre-operation adjustment user data; and
post-operation adjustment user data.

13. A method (200) for adjusting a manner in which a system interacts with a target user and a support user, the support user being a support provider of the target user, the method comprising:
monitoring (210) the target user and/or the support user and/or receiving an input from the target user and/or the support user, thereby obtaining user data relating to a mental state of the target user and/or the support user, for example a behavioral state or a psychological state of the target user and/or the support user;
operating (220) the system in an initial operation mode, the initial operation mode having a mode type;
determining (230), based on the user data and the mode type, an operation mode type adjustment; and
applying (240) the operation mode adjustment to the interactive user system.

14. A method (200) as claimed in claim 13, wherein the method further comprises obtaining user feedback, wherein the user feedback relates to a subjective user experience based on the adjusted operation mode.

15. A computer program comprising computer program code means which is adapted, when said computer program is run on a computer, to implement the method of any of claims 13 to 14.
